# EUROPÄISCHE PATENTSCHRIFT

(11) **EP 1 904 430 B1**
(45) Veröffentlichungstag und Bekanntmachung des Hinweises auf die Patenterteilung: **11.09.2013**
(21) Anmeldenummer: 06777711.0
(22) Anmeldetag: 11.07.2006
(51) Int. Cl.: C07C 45/62, C07C 45/85, C07C 49/413, C07C 47/228, C07B 35/02

(54) **VERFAHREN ZUR HYDRIERUNG VON ALDEHYDHALTIGEN STOFFSTRÖMEN**
METHOD FOR THE HYDROGENATION OF MASS FLUXES CONTAINING ALDEHYDE
PROCEDE D'HYDROGENATION DE FLUX DE MATIERES CONTENANT DES ALDEHYDES

(30) Priorität: 12.07.2005 DE 102005032541
(43) Veröffentlichungstag der Anmeldung: 02.04.2008
(73) Patentinhaber: BASF SE, 67056 Ludwigshafen (DE)
(72) Erfinder: PINKOS, Rolf, 67098 Bad Dürkheim (DE); TEBBEN, Gerd-Dieter, 68239 Mannheim (DE); HAUK, Alexander, 67067 Ludwigshafen (DE); MÜLLER, Christian, 68167 Mannheim (DE); RUST, Harald, 67435 Neustadt (DE)
(86) Internationale Anmeldenummer: PCT/EP2006/064115
(87) Internationale Veröffentlichungsnummer: WO 2007/006789

(56) Entgegenhaltungen:
- WO-A-01/27061
- DE-A1- 10 344 595
- GB-A- 1 519 677
- US-A- 4 213 000
- US-A- 4 960 960

## Beschreibung

Die vorliegende Erfindung betrifft ein Verfahren zur Hydrierung einer Zusammensetzung I, enthaltend mindestens einen Aldehyd und Cyclododecadienon, das durch Umsetzung eines Cyclododecatriens mit Distickstoffmonoxid erhalten wurde, mit Wasserstoff in Gegenwart eines Katalysators in mindestens einem Hauptreaktor und mindestens einem Nachreaktor, wobei mindestens 50% des dem Reaktionssystem zugeführten Frischwasserstoffs in mindestens einen Nachreaktor eingespeist werden und wobei mindestens 70% des Abgases des Hauptreaktors ausgeschleust werden.

Hydrierungen von aldehydhaltigen Zusammensetzungen sind weit verbreitet Beispielsweise werden bei der Herstellung von sogenannten Oxoalkoholen nach Hydroformylierung von Olefinen zu Aldehyden diese zu Alkoholen hydriert (K. Weissermel, H.J. Arpe, "Industrielle Organische Chemie" 5. Auflage 1998, Seiten 149 - 150, Wiley-VCH Verlag).

Die US 4,960,960 offenbart die Hydrierung ungesättigter organischer Verbindungen, beispielsweise von Aldehyden oder 1,4-Butindiol. Insbesondere offenbart die US 4,960,960 die Umsetzung in einem Haupt- und einem Nachreaktor, wobei der Frischwasserstoff dem Nachreaktor zugeführt wird.

GB 1 519 677 A offenbart ein Verfahren zur Hydrierung organischer Verbindungen, beispielsweise 1,4-Diacetoxybuten, in Gegenwart eines Aldehyds, wobei 50 % des Frischwasserstoffs dem Nachreaktor zugeführt werden und das Abgas aus dem Hauptreaktor ausgeschleust wird.

Beispielsweise wird Lysmeral durch Hydrierung von Dehydrolysmeral, gegebenenfalls in Gegenwart von tert.Butylbenzaldehyd, hergestellt, wie in WO 01/27061 beschrieben.

Ebenso sind Verfahren bekannt, bei denen eine organische Verbindung hydriert wird, die als Verunreinigung einen Aldehyd enthält. Als Beispiel ist die Hydrierung von Cyclododecadienon zu Cyclododecanon zu nennen, wobei geringe Mengen Aldehyde im Cyclododecadienon enthalten sein können. Ein derartiges Verfahren ist beispielsweise in DE 103 44 595 A beschrieben.

Ebenso können bei der Hydrierung von Butindiol zu Butandiol geringe Mengen Formaldehyd aus der Synthese des Butindiols aus Formaldehyd und Acetylen anwesend sein. Es kann aber auch gezielt Formaldehyd noch zugesetzt werden, um alkyliertes Butandiol zu erhalten, wie beispielsweise in EP 0 133 739 B1 offenbart.

Bei diesen Hydrierverfahren kommt es durch Zersetzung von im Reaktionssystem vorhandenen Aldehyden zur Bildung von CO, von dem bekannt ist, dass es die Hydrierung behindern kann. Dies ist insbesondere dann ein Problem, wenn ein vollständiger Umsatz der Edukte benötigt wird, beispielsweise, um Produkte in hoher Reinheit zu erhalten oder aufwendige Trennverfahren zu vermeiden. Dem Problem wird oft dadurch begegnet, dass bei sehr hohen Drücken hydriert wird, um entstehendes CO möglichst vollständig zu Methan umzusetzen. Der benötigte hohe Druck führt jedoch dazu, dass hohe Investitions- und Betriebskosten für das Verfahren anfallen.

Um einen möglichst vollständigen Umsatz zu erreichen, ist es im Allgemeinen üblich, in zwei Schritten, einem sogenannten Hauptreaktor und mindestes einem Nachreaktor, zu hydrieren. Dabei wird im Hauptreaktor der größte Teil der Hydrierwärme frei und durch äußeren Umlauf abgeführt. Der Frischwasserstoff wird üblicherweise zumindest zum größten Teil in den Hauptreaktor eingebracht und das Abgas aus oder nach dem letzten Nachreaktor ausgeschleust.

Bei einer derartigen Verfahrensführung liegen insbesondere im Nachreaktor hohe CO-Konzentrationen vor und es wird unter Umständen kein vollständiger Umsatz erreicht. Damit werden Produkte erhalten, die Nebenkomponenten enthalten, die teilweise nur durch aufwendige und kostspielige Reinigungsschritte abgetrennt werden können.

Demgemäß lag der vorliegenden Erfindung die Aufgabe zugrunde, ein Verfahren zur Hydrierung aldehydhaltiger Zusammensetzungen bereitzustellen, wobei ein möglichst hoher Umsatz der Edukte erreicht wird.

Eine weitere Aufgabe der vorliegenden Erfindung war es, ein Verfahren zur Hydrierung aldehydhaltiger Zusammensetzungen bereitzustellen, bei dem Produkte in hoher Reinheit erhalten werden.

Eine weitere Aufgabe der vorliegenden Erfindung war es, ein kostengünstiges Verfahren zur Hydrierung aldehydhaltiger Zusammensetzungen bereitzustellen, bei dem Produkte in hoher Reinheit erhalten werden.

Erfindungsgemäß wird diese Aufgabe gelöst durch ein Verfahren zur Hydrierung einer Zusammensetzung I, enthaltend mindestens einen Aldehyd und Cyclododecadienon, das durch Umsetzung eines Cyclododecatriens mit Distickstoffmonoxid erhalten wurde, mit Wasserstoff in Gegenwart eines Katalysators in mindestens einem Hauptreaktor und mindestens einem Nachreaktor, wobei mindestens 50% des dem Reaktionssystem zugeführten Frischwasserstoffs in mindestens einen Nachreaktor eingespeist werden und wobei mindestens 70% des Abgases des Hauptreaktors ausgeschleust werden.

Die vorliegenden Erfindung betrifft gemäß einer bevorzugten Ausführungsform ein wie zuvor beschriebenes Verfahren, zur Hydrierung einer Zusammensetzung I wobei mehr als 50 % des in mindestens einem Hauptreaktor eingesetzten Wasserstoffs aus dem Abgas des Nachreaktors stammen.

Unter "Frischwasserstoff" wird im Rahmen der vorliegenden Erfindung Wasserstoff oder ein wasserstoffhaltiges Gemisch verstanden, das dem Reaktionssystem zugeführt wird.

Im Rahmen der vorliegenden Anmeldung wird unter dem Begriff "Reaktionssystem" die Summe der für das erfindungsgemäße Verfahren genutzten Reaktoren verstanden, also mindestens ein Hauptreaktor und mindestens ein Nachreaktor.

Es wurde gefunden, dass es besonders vorteilhaft ist, wenn der dem Reaktionssystem zugesetzte Wasserstoff, zumindest zum größten Teil, in den Nachreaktor eingebracht wird und von dort als Abgas des Nachreaktors in den Hauptreaktor überführt wird. Erfindungsgemäß wird der größte Teil des Abgases aus oder nach dem Hauptreaktor ausgeschleust. In den Nachreaktor gelangt aus dem Hauptreaktor erfindungsgemäß vorzugsweise nur der im Produktstrom des Hauptreaktors gelöste Wasserstoff.

Das erfindungsgemäße Verfahren hat unter anderem den Vorteil, dass im Nachreaktor eine geringe CO-Konzentration vorliegt. Durch diese Maßnahme gelingt es, den Umsatz zu maximieren, ohne auf hohe Drücke zurückgreifen zu müssen. Somit kann beim erfindungsgemäßen Verfahren Katalysator und Reaktorraum eingespart werden. Ein weiterer Vorteil ist die erhöhte Lebensdauer der Katalysatoren, insbesondere in dem oder den Nachreaktoren.

Im Allgemeinen wird bei Hydrierungen, bei denen Vollumsatz angestrebt wird, mit Wasserstoff-Überschuss hydriert. Das erfindungsgemäße Verfahren zeichnet sich dadurch aus, dass der Wasserstoff-Überschuss, der zwangsläufig entsorgt werden muss und damit Kosten verursacht, relativ gering sein kann. Bevorzugt liegt der Wasserstoffüberschuss (Mol-% Wasserstoff bezogen auf den stöchiometrisch benötigten Wasserstoff) zwischen 0,1 und 50 Mol-%. Besonders bevorzugt zwischen 0,3 und 40 Mol-%, ganz besonders bevorzugt zwischen 0,5 und 25 Mol-%.

Daher betrifft die vorliegende Erfindung gemäß einer bevorzugten Ausführungsform auch ein wie zuvor beschriebenes Verfahren, wobei Wasserstoff in einem Überschuss von 0,1 bis 50 Mol-% eingesetzt wird.

Das erfindungsgemäße Verfahren ist anwendbar für die hydrierung einer Zusammensetzung I. Gemäß einer bevorzugten Ausführungsform kann die Zusammensetzung I neben dem Aldehyd und Cyclododecadienon auch mindestens eine weitere organische Verbindung enthalten, die in dem erfindungsgemäßen Verfahren hydriert wird. Bei der organischen Verbindung handelt es sich also im Rahmen der vorliegenden Erfindung um eine hydrierbare organische Verbindung..

Die vorliegende Erfindung betrifft demgemäß auch ein wie zuvor beschriebenes Verfahren zur Hydrierung einer Zusammensetzung I, mit Wasserstoff in Gegenwart eines Katalysators in mindestens einem Hauptreaktor und mindestens einem Nachreaktor, wobei die Zusammensetzung **I** mindestens eine weitere organische Verbindung enthält.

Sofern der Aldehyd die zu hydrierende Hauptkomponente der Zusammensetzung I ist, liegt der Aldehydgehalt im Rahmen der vorliegenden Erfindung vorzugsweise bei mindestens 80 % und höchstens 100 %, bevorzugt mindestens 90 % und höchstens 99,9 %, besonders bevorzugt mindestens 95 %, ganz besonders bevorzugt mindestens 98 % und höchstens 99,5 %.

Sofern Aldehyde als Nebenkomponenten vorhanden sind, enthält die Zusammensetzung I im Rahmen der vorliegenden Erfindung insbesondere 20 bis 99,9 Gew.%, bezogen auf die Zusammensetzung I, einer weiteren organischen Verbindung. Vorzugsweise enthält die Zusammensetzung I 70 bis 98 Gew.-%, besonders bevorzugt 90 bis 95 Gew.% einer weiteren organischen Verbindung.

Neben organischen Bestandteilen kann die Zusammensetzung I auch anorganische Verbindungen, wie z.B. Wasser, enthalten.

Als organische Verbindung können im Rahmen der vorliegenden Erfindung alle geeigneten Verbindungen eingesetzt werden, die mit Wasserstoff in Gegenwart eines Katalysators hydriert werden können. Erfindungsgemäß geeignet sind beispielsweise ungesättigte, gegebenenfalls substituierte Verbindungen wie Alkene oder Alkine, aromatische Verbindungen, Ketone, Ester, Säuren, Anhydride, Amide, Nitrile oder Nitroverbindungen.

Erfindungsgemäß können insbesondere organische Verbindungen eingesetzt werden, die beispielsweise bedingt durch das Herstellungsverifahren Aldehyde enthalten. Derartige Verbindungen sind beispielsweise Dehydrolysmeral oder 1,4-Butindiol.

Demgemäß betrifft die vorliegende Erfindung gemäß einer weiteren Ausführungsform ein wie zuvor beschriebenes Verfahren zur Hydrierung einer Zusammensetzung I, enthaltend mindestens einen Aldehyd und Cyclododecadienon mindestens eine weitere organische Verbindung, mit Wasserstoff in Gegenwart eines Katalysators in mindestens einem Hauptreaktor und mindestens einem Nachreaktor, wobei die organische Verbindung ausgewählt ist aus der Gruppe bestehend aus Dehydrolysmeral oder 1,4-Butindiol.

Die Zusammensetzung I enthält Cyclododecadienon, das durch Umsetzung eines Cyclododecatriens mit Distickstoffmonoxid erhalten wurde.

Dabei kann für die Umsetzung des Cyclododecatriens mit Distickstoffmonoxid mindestens ein geeignetes Lösungsmittel oder Verdünnungsmittel eingesetzt werden. Als solche sind unter anderem Cyclododecan oder Cyclododecanon oder gesättigte aliphatische oder aromatische, gegebenenfalls alkylsubstituierte Kohlenwasserstoffe zu nennen, wobei im Wesentlichen alle gängigen Lösungs- und/oder Verdünnungsmittel geeignet sind unter der Maßgabe, dass sie weder eine C-C-Doppelbindung, noch eine C-C-Dreifachbindung, noch eine Aldehydgruppe aufweisen.

Im Allgemeinen ist bei der Umsetzung von Cyclododecatrien mit Distickstoffmonoxid der Zusatz eines Lösungsmittels oder Verdünnungsmittel nicht notwendig.

Die Temperaturen bei der Umsetzung von Cyclododecatrien mit Distickstoffmonoxid liegen bevorzugt im Bereich von 140 bis 350 °C, weiter bevorzugt im Bereich von 180 bis 320 °C und besonders bevorzugt im Bereich von 200 bis 300 °C.

Es ist möglich, die Umsetzung von Cyclododecatrien mit Distickstoffmonoxid bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden.

Die Drücke bei der Umsetzung von Cyclododecatrien mit Distickstoffmonoxid liegen bevorzugt höher als der Eigendruck des Edukt- bzw. Produktgemisches bei der gewählten Reaktionstemperatur oder den gewählten Reaktionstemperaturen. Die Drücke liegen bevorzugt im Bereich von 1 bis 1000 bar, weiter bevorzugt im Bereich von 40 bis 300 bar und besonders bevorzugt im Bereich von 50 bis 200 bar.

Es ist möglich, die Umsetzung von Cyclododecatrien mit Distickstoffmonoxid bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden.

Hinsichtlich der für die Umsetzung von Cyclododecatrien mit Distickstoffmonoxid einsetzbaren Reaktoren existieren keine besonderen Beschränkungen. Insbesondere kann die Umsetzung in Batch-Fahrweise oder in kontinuierlicher Fahrweise erfolgen. Demgemäß können beispielsweise als Reaktoren mindestens ein CSTR (Continuous Stirred Tank Reactor) mit mindestens einem internen und/oder mindestens einem externen Wärmetauscher, mindestens ein Rohrreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Bevorzugt wird die Umsetzung von Cyclododecatrien mit Distickstoffmonoxid in einem einzigen Reaktor durchgeführt. Beispielsweise bevorzugt ist die Umsetzung in kontinuierlicher Fahrweise.

Die Verweilzeit des Reaktionsgutes in dem mindestens einen Reaktor bei der Umsetzung von Cyclododecatrien mit Distickstoffmonoxid liegt im allgemeinen im Bereich von bis zu 20 h, bevorzugt im Bereich von 0,1 bis 20 Stunden, weiter bevorzugt im Bereich von 0,2 bis 15 Stunden und besonders bevorzugt im Bereich von 0,25 bis 10 h.

Im Feed, der der Umsetzung von Distickstoffmonoxid mit Cyclododecatrien zugeführt wird, liegt das Molverhältnis von Distickstoffmonoxid zu Cyclododecatrien im Allgemeinen im Bereich von 0,05 bis 4, bevorzugt im Bereich von 0,06 bis 1, weiter bevorzugt im Bereich von 0,07 bis 0,5 und besonders bevorzugt im Bereich von 0,1 bis 0,4.

Die Umsetzung von Cyclododecatrien mit Distickstoffmonoxid kann so durchgeführt werden, dass bei einer sehr hohen Selektivität bezüglich Cyclododecadienon ein Umsatz von Cyclododecatrien im Bereich von bis zu 50 %, bevorzugt im Bereich von 5 bis 30 % und insbesondere bevorzugt im Bereich von 10 bis 20 % erreicht wird. Dabei liegt die Selektivität, bezogen auf Cyclododecadienon, im Allgemeinen bei mindestens 90 %, bevorzugt bei mindestens 92,5 % und besonders bevorzugt bei mindestens 95 %.

Grundsätzlich kann jedes Cyclododecatrien oder jedes Gemisch aus zwei und mehr unterschiedlichen Cyclododecatrienen mit Distickstoffmonoxid umgesetzt werden. Unter anderem sind hierbei beispielsweise 1,5,9-Cyclododecatriene, beispielsweise cis,trans,trans-1,5,9-Cyclododecatrien oder cis,cis,trans-1,5,9-Cyclododecatrien oder all-trans-1,5,9-Cyclododecatrien, zu nennen.

Bevorzugt wird als Cyclododecatrien cis,trans,trans-1,5,9-Cyclododecatrien eingesetzt.

Im allgemeinen resultiert aus der Umsetzung von cis,trans,trans-1,5,9-Cyclododecatrien mit Distickstoffmonoxid ein Cyclododeca-4,8-dienon-Isomerengemisch, das mindestens zwei der Isomere cis,trans-Cyclododeca-4,8-dienon, trans,cis-Cyclododeca-4,8-dienon und trans,trans-Cyclododeca-4,8-dienon enthält. Ein beispielsweise typisches Isomerengemisch weist demgemäß die Isomeren in molaren Verhältnissen von in etwa 1 : 1 : 0,08 auf. Dies Isomerengemisch kann in der im erfindungsgemäßen Verfahren eingesetzten Zusammensetzung I enthalten sein.

Die Umsetzung von Cyclododecatrien mit Distickstoffmonoxid kann grundsätzlich in Anwesenheit eines Katalysators erfolgen, jedoch auch ohne Zusatz eines Katalysators.

1,5,9-Cyclododecatrien kann beispielsweise durch Trimerisierung von reinem 1,3-Butadien hergestellt werden, wie dies beispielsweise in T. Schiffer, G. Oenbrink, "Cyclododecatriene, Cyclooctadiene, and 4-Vinylcyclohexene", Ullmann's Encyclopedia of Industrial Chemistry, 6th Edition (2000), Electronic Release, Wiley VCH beschrieben ist. Im Rahmen dieses Verfahrens entstehen beispielsweise bei der Trimerisierung in Anwesenheit von Ziegler-Katalysatoren cis,trans,trans-1,5,9-Cyclododecatrien, cis,cis,trans-1,5,9-Cyclododecatrien und all-trans-1,5,9-Cyclododecatrien, wie dies beispielsweise in H. Weber et al. "Zur Bildungsweise von cis,trans,trans-Cyclododecatrien-(1.5.9) mittels titanhaltiger Katalysatoren" in: Liebigs Ann. Chem. 681 (1965) S.10-20 beschrieben ist. Cyclododecatrien kann durch Trimerisierung von 1,3-Butadien unter Verwendung eines Titan-Katalysators hergestellt werden.

Während grundsätzlich sämtliche geeigneten Titan-Katalysatoren zur Trimerisierung eingesetzt werden können, ist der im Artikel von Weber et al. beschriebene Titantetrachlorid/Ethylaluminiumsesquichlorid-Katalysator besonders geeignet.

Das für die Trimerisierung eingesetzte Butadien weist insbesondere bevorzugt einen über Gaschromatographie bestimmten Reinheitsgrad von mindestens 99,6 % und weiter bevorzugt von mindestens 99,65 % auf. Insbesondere bevorzugt enthält das eingesetzte 1,3-Butadien im Rahmen der Nachweisgenauigkeit kein 1,2-Butadien und kein 2-Butin.

Aus dieser Trimerisierung werden im Allgemeinen Gemische erhalten, die mindestens 95 Gew.%, bevorzugt mindestens 96 Gew.-% und weitere bevorzugt mindestens 97 Gew.% cis,trans,trans-1,5,9-Cyclododecatrien enthalten. Beispielsweise insbesondere bevorzugt enthalten die Gemische in etwa 98 Gew.-% cis,trans,trans-1,5,9-Cyclododecatrien.

Dieses cis,trans,trans-1,5,9-Cyclododecatrien enthaltende Gemisch kann als solches für die Umsetzung mit Distickstoffmonoxid verwendet werden. Ebenso ist es möglich, über mindestens eine geeignete Methode, beispielsweise bevorzugt über mindestens eine Destillation, das cis,trans,trans-1,5,9-Cyclododecatrien aus dem Gemisch abzutrennen und in der Umsetzung mit Distickstoffmonoxid einzusetzen.

Erfindungsgemäß ist in der Zusammensetzung I ein Aldehyd und Cyclododecadienon enthalten. Das erfindungsgemäße Verfahren kann grundsätzlich in Gegenwart jedes beliebigen Aldehyds durchgeführt werden. Es kann sich dabei beispielsweise um Aldehyde mit 1 bis 20 C-Atomen handeln.

Die vorliegende Erfindung betrifft demgemäß in einer weiteren Ausführungsform ein wie zuvor beschriebenes Verfahren zur Hydrierung einer Zusammensetzung I, wobei der mindestens eine Aldehyd ausgewählt ist aus der Gruppe bestehend aus C1- bis C20-Adehyden.

Die Zusammensetzung I enthält im Rahmen der vorliegenden Erfindung insbesondere 0,1 bis 80 Gew.-%, bezogen auf die Zusammensetzung I, des Aldehyds. Vorzugsweise enthält die Zusammensetzung I 2 bis 30 Gew.%, besonders bevorzugt 5 bis 10 Gew.-% des Aldehyds.

Das erfindungsgemäße Verfahren wird in mindestens einem Hauptreaktor und mindestens einem Nachreaktor durchgeführt. Dabei werden erfindungsgemäß vorzugsweise mindestens 60% der Zusammensetzung I im Hauptreaktor umgesetzt.

Weiter bevorzugt werden im erfindungsgemäßen Verfahren mindestens 85% der Zusammensetzung I im Hauptreaktor umgesetzt, besonders bevorzugt werden mindestens 90% der Zusammensetzung I im Hauptreaktor umgesetzt.

Demgemäß betrifft die vorliegende Erfindung gemäß einer bevorzugten Ausführungsform ein wie zuvor beschriebenes Verfahren zur Hydrierung einer Zusammensetzung I, wobei im Hauptreaktor mindestens 60% der Zusammensetzung I umgesetzt werden.

Erfindungsgemäß werden mindestens 50 % des dem Reaktionssystem zugeführten Frischwasserstoffs in mindestens einen Nachreaktor eingespeist Gemäß einer weiter bevorzugten Ausführungsform werden beispielsweise mindestens 60% des Frischwasserstoffs in den Nachreaktor eingespeist, insbesondere mindestens 70%, ganz besonders bevorzugt mindestens 80% und insbesondere bevorzugt mindestens 90%.

Demgemäß werden im Rahmen der vorliegenden Erfindung insbesondere 60 bis 100 % des dem Reaktionssystem zugeführten Frischwasserstoffs in mindestens einen Nachreaktor eingespeist, bevorzugt 70 bis 99%, besonders bevorzugt 80 bis 95%.

Die vorliegende Erfindung betrifft demgemäß in einer weiteren Ausführungsform ein wie zuvor beschriebenes Verfahren zur Hydrierung einer Zusammensetzung I, wobei mindestens 60% des dem Reaktionssystem zugeführten Frischwasserstoffs in mindestens einen Nachreaktor eingespeist werden.

Erfindungsgemäß ist es ebenfalls möglich, dass dem Reaktionssystem weiterer Wasserstoff, beispielsweise über mindestens einen Hauptreaktor oder mehrere Hauptreaktoren zugeführt wird.

Erfindungsgemäß kann das bei dem erfindungsgemäßen Verfahren anfallende Abgas überwiegend aus oder nach mindestens einem Hauptreaktor ausgeschleust werden. So werden mindestens 70% des Abgases des Hauptreaktors ausgeschleust, bevorzugt mindestens 75%, besonders bevorzugt mindestens 80%, insbesondere bevorzugt mindestens 90%. Bevorzugt gelangt abgesehen von dem gelösten Teil des Wasserstoffs kein Gas von dem oder den Hauptreaktor(en) in den oder die Nachreaktoren.

Ebenso ist es im Rahmen der vorliegenden Erfindung möglich, dass ein Teil des Abgases des Nachreaktors ausgeschleust wird, insbesondere sofern gewährleistet ist, dass mindestens 50% des in mindestens einem Hauptreaktor eingesetzten Wasserstoffs aus dem Abgas des Nachreaktors stammen. Vorzugsweise stammen im Rahmen der vorliegenden Erfindung mindestens 60% des in mindestens einem Hauptreaktor eingesetzten Wasserstoffs aus dem Abgas des Nachreaktors, besonders bevorzugt mindestens 70%, ganz besonders bevorzugt mindestens 80%.

Demgemäß betrifft die vorliegende Erfindung auch gemäß einer bevorzugten Ausführungsform ein wie zuvor beschriebenes Verfahren zur Hydrierung einer Zusammensetzung I, wobei Wasserstoff nur in Form von im Produktstrom des Hauptreaktors gelöstem Wasserstoff von mindestens einem Hauptreaktor zu mindestens einem Nachreaktor gelangt.

Gemäß dem erfindungsgemäßen Verfahren wird das bei der Umsetzung in einem Hauptreaktor erhaltene Gemisch in einen weiteren Hauptreaktor oder einen Nachreaktor zur weiteren Umsetzung überführt. Das Produkt der Umsetzung der Zusammensetzung I mit Wasserstoff wird im Rahmen der vorliegenden Erfindung aus dem Nachreaktor entnommen.

Für die Hydrierung der Zusammensetzung I können sämtliche geeigneten Katalysatoren eingesetzt werden, Insbesondere sind mindestens ein homogener oder mindestens ein heterogener oder sowohl mindestens ein homogener und mindestens ein heterogener Katalysator einsetzbar.

Bevorzugt enthalten die einsetzbaren Katalysatoren mindestens ein Metall aus der 7., der 8., der 9., der 10. oder der 11. Nebengruppe des Periodensystems der Elemente. Weiter bevorzugt enthalten die erfindungsgemäß einsetzbaren Katalysatoren mindestens ein Element, ausgewählt aus der Gruppe bestehend aus Re, Fe, Ru, Co, Rh, Ir, Ni, Pd, Pt, Cu und Au. Insbesondere bevorzugt enthalten die erfindungsgemäß einsetzbaren Katalysatoren mindestens ein Element, ausgewählt aus der Gruppe bestehend aus Fe, Ni, Pd, Pt und Cu. Besonders bevorzugt enthalten die erfindungsgemäß einsetzbaren Katalysatoren Pd, Pt, Ru oder Ni.

Die vorliegende Erfindung betrifft daher gemäß einer weiteren Ausführungsform ein wie zuvor beschriebenes Verfahren zur Hydrierung einer Zusammensetzung I, wobei der Katalysator als Aktivmetall Pd, Pt, Ru oder Ni enthält.

Im erfindungsgemäßen Verfahren bevorzugt eingesetzte homogene Katalysatoren enthalten mindestens ein Element der 8., 9. oder 10. Nebengruppe. Weiter bevorzugt sind homogene Katalysatoren, die Ru, Rh, Ir und/oder Ni enthalten. Beispielsweise sind hierbei etwa RhCl(TTP)₃ oder Ru₄H₄(CO)₁₂ zu nennen. Besonders bevorzugt sind solche homogenen Katalysatoren, die Ru enthalten. Beispielsweise werden homogene Katalysatoren eingesetzt, wie sie in der US 5,180,870, US 5,321,176, US 5,177,278, US 3,804,914, US 5,210,349 US 5,128,296, US B 316,917 und in D.R. Fahey in J. Org. Chem. 38 (1973) S. 80-87 beschrieben sind, deren diesbezügliche Offenbarung in den Kontext der vorliegenden Anmeldung vollumfänglich einbezogen wird. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)₂RuH₂, (TPP)₂(CO)₂RuClH oder (TPP)₃(CO)RuCl₂.

Insbesondere bevorzugt wird im Rahmen des erfindungsgemäßen Verfahrens mindestens ein heterogener Katalysator eingesetzt, wobei mindestens eines der obenstehend genannten Metalle als Metall als solches, als Raney-Katalysator und/oder aufgebracht auf einen üblichen Träger eingesetzt werden kann. Bevorzugte Trägermaterialien sind etwa Aktivkohlen oder Oxide wie beispielsweise Aluminiumoxide, Siliciumoxide, Titanoxide oder Zirkonoxide. Ebenso sind unter anderem als Trägermaterialien Bentonite zu nennen. Werden zwei oder mehr Metalle eingesetzt, so können diese separat oder als Legierung vorliegen. Hierbei ist es möglich, mindestens ein Metall als solches und mindestens ein anderes Metall als Raney-Katalysator oder mindestens ein Metall als solches und mindestens ein anderes Metall, aufgebracht auf mindestens einen Träger, oder mindestens ein Metall als Raney-Katalysator und mindestens ein anderes Metall, aufgebracht auf mindestens einen Träger, oder mindestens ein Metall als solches und mindestens ein anderes Metall als Raney-Katalysator und mindestens ein anderes Metall, aufgebracht auf mindestens einen Träger, einzusetzen.

Die im erfindungsgemäßen Verfahren eingesetzten Katalysatoren können beispielsweise auch so genannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, beispielsweise schwerlöslichen Hydroxiden, Oxidhydraten, basischen Salzen oder Carbonaten ausfällt, die erhaltenen Niederschläge anschließend trocknet und diese dann durch Calcinierung bei im allgemeinen 300 bis 700 °C, insbesondere 400 bis 600 °C in die entsprechenden Oxide, Mischoxide und/oder gemischtvalentigen Oxide umwandelt, die durch eine Behandlung mit Wasserstoff oder mit Wasserstoff enthaltenden Gasen im Bereich von im Allgemeinen 50 bis 700 °C, insbesondere 100 bis 400 °C zu den betreffenden Metallen und/oder oxidischen Verbindungen niederer Oxidationsstufe reduziert und in die eigentliche katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten.

Außer den oben genannten Fällungskatalysatoren, die außer den katalytisch aktiven Komponenten noch zusätzlich ein Trägermaterial enthalten, eignen sich für das erfindungsgemäße Verfahren im Allgemeinen solche Trägermaterialien, bei denen die katalytisch-hydrierend wirkende Komponente beispielsweise durch Imprägnierung auf ein Trägermaterial aufgebracht worden ist.

Die Art des Aufbringens des katalytisch aktiven Metalls auf den Träger ist in der Regel nicht kritisch und kann auf verschiedenerlei Art und Weise bewerkstelligt werden. Die katalytisch aktiven Metalle können auf diesen Trägermaterialien beispielsweise durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, beispielsweise mit Nitraten oder thermisch leicht zersetzbaren Komplexverbindungen, beispielsweise Carbonyl- oder Hydrido-Komplexen der katalytisch aktiven Metalle, zu imprägnieren und den so getränkten Träger zur thermischen Zersetzung der adsorbierten Metallverbindungen auf Temperaturen im Bereich von 300 bis 600 °C zu erhitzen. Diese thermische Zersetzung wird vorzugsweise unter einer Schutzgasatmosphäre vorgenommen. Geeignete Schutzgase sind beispielsweise Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Im Allgemeinen führen höhere Gehalte an katalytisch aktiven Metallen dieser Trägerkatalysatoren zu höheren Raum-Zeit-Umsätzen als niedrigere Gehalte. Im Allgemeinen werden Trägerkatalysatoren verwendet, deren Gehalt an katalytisch aktiven Metallen im Bereich von 0,1 bis 90 Gew.-%, vorzugsweise im Bereich von 0,5 bis 40 Gew.% bezogen auf das Gesamtgewicht des Katalysators, liegt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, ist es auch denkbar, dass diese Angaben auch unter- oder überschritten werden können, ohne dass sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein.

Weiterhin können die katalytisch aktiven Metalle beispielsweise nach dem Verfahren von DE-OS 25 19 817, EP 1 477 219 A1 oder EP 0 285 420 A1 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung mit einem Salz oder Komplex der zuvor genannten Metalle, erzeugt werden.

Sowohl die Aktivierung der Fällungskatalysatoren als auch der Trägerkatalysatoren kann auch in situ zu Beginn der Reaktion durch den anwesenden Wasserstoff erfolgen. Bevorzugt werden diese Katalysatoren vor ihrer Verwendung separat aktiviert.

Als Trägermaterialien können im Allgemeinen die Oxide des Aluminiums und Titans, Zirkoniumdioxid, Siliciumdioxid, Tonerden wie beispielsweise Montmorillonite, Silikate wie beispielsweise Magnesium- oder Aluminiumsilikate, Zeolithe wie beispielsweise der Strukturtypen ZSM-5 oder ZSM-10, oder Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren verwendbare Katalysatoren dienen.

Ganz besonders bevorzugte Katalysatoren sind erfindungsgemäß solche, die Ni, Pt und/oder Pd enthalten und auf einem Träger aufgebracht sind. Ganz bevorzugte Träger sind oder enthalten Aktivkohle, Aluminiumoxid, Titandioxid und/oder Siliziumdioxid.

In dem oder den Haupt- und Nachreaktoren kann der gleiche Katalysator vorliegen, dies ist aber im Rahmen der vorliegenden Erfindung nicht zwingend. Es kann beispielsweise auch in jedem der Reaktoren ein anderer Katalysator eingesetzt werden. So kann beispielsweise eine Suspensionshydrierung mit homogener Hydrierung und/oder heterogener Hydrierung kombiniert sein. Besonders bevorzugt ist die Hydrierung mit heterogenen Katalysatoren, wobei insbesondere der in dem oder den Nachreaktoren eingesetzte Katalysator als Festbett verwendet wird.

Der mindestens eine heterogene Katalysator kann beispielsweise als Suspensionskatalysator und/oder als Festbettkatalysator eingesetzt werden.

Wird beispielsweise im Rahmen des erfindungsgemäßen Verfahrens die Hydrierung mit mindestens einem Suspensionskatalysator durchgeführt, so wird bevorzugt in mindestens einem Rührreaktor oder in mindestens einer Blasensäule oder in mindestens einer gepackten Blasensäule oder in einer Kombination aus zwei oder mehr gleichen oder unterschiedlichen Reaktoren hydriert.

Der Begriff "unterschiedliche Reaktoren" bezeichnet vorliegend sowohl unterschiedliche Reaktortypen als auch Reaktoren der gleichen Art, die sich beispielsweise durch ihre Geometrie wie beispielsweise ihr Volumen und/oder ihren Querschnitt und/oder durch die Hydrierbedingungen in den Reaktoren unterscheiden.

Wird beispielsweise im Rahmen des erfindungsgemäßen Verfahrens die Hydrierung mit mindestens einem fest angeordneten Katalysator durchgeführt, so wird bevorzugt mindestens ein Rohrreaktor wie beispielsweise mindestens ein Schachtreaktor und/oder mindestens ein Rohrbündelreaktor verwendet, wobei ein einzelner Reaktor in Sumpf- oder Rieselfahrweise betrieben werden kann. Bei Verwendung von zwei oder mehr Reaktoren kann mindestens einer in Sumpffahrweise und mindestens einer in Rieselfahrweise betrieben werden.

Gemäß einer bevorzugten Ausführungsform des erfindungsgemäßen Verfahrens wird der für die Hydrierung eingesetzte mindestens eine Katalysator nach der Umsetzung in einem Hauptreaktor aus dem Produktgemisch der Hydrierung abgetrennt. Diese Abtrennung kann in Abhängigkeit vom eingesetzten Katalysator gemäß jeder geeigneten Verfahrensführung erfolgen.

Ebenso kann eine Abtrennung eines Katalysators nach einem Nachreaktor durch geeignete Verfahrensführung erfolgen. Die nachfolgenden Ausführungen betreffen sowohl die Katalysatorabtrennung nach einem Haupt- oder einem Nachreaktor.

Wird als Katalysator bei der Hydrierung beispielsweise ein heterogener Katalysator als Suspensionskatalysator eingesetzt, so wird dieser im Rahmen der vorliegenden Erfindung bevorzugt durch mindestens einen Filtrationsschritt abgetrennt. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurück zu gewinnen.

Wird als Katalysator bei der Hydrierung beispielsweise ein homogener Katalysator eingesetzt, so wird dieser im Rahmen der vorliegenden Erfindung bevorzugt durch mindestens einen Destillationsschritt abgetrennt. Im Rahmen dieser Destillation können eine oder zwei oder mehr Destillationskolonnen verwendet werden. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurück zu gewinnen.

Vor dem Einsatz in einem beliebigen Verfahren wie beispielsweise vor der Rückführung in das erfindungsgemäße Verfahren kann sowohl der mindestens eine homogene als auch der mindestens eine heterogene Katalysator, sollte dies erforderlich sein, durch mindestens ein geeignetes Verfahren regeneriert werden.

Die Wärme kann bei dem erfindungsgemäß verwendeten Reaktor intern beispielsweise über Kühlschlangen und/oder extern beispielsweise über mindestens einen Wärmetauscher abgeführt werden. Wird beispielsweise bevorzugt mindestens ein Rohrreaktor zur Hydrierung eingesetzt, so wird bevorzugt die Reaktion über äußeren Umlauf gefahren, in dem die Wärmeabfuhr integriert ist.

Wird gemäß einer bevorzugten Ausführungsform des erfindungsgemäßen Verfahrens die Hydrierung kontinuierlich durchgeführt, werden weiter bevorzugt mindestens zwei Reaktoren, weiter bevorzugt mindestens zwei Rohrreaktoren, weiter bevorzugt mindestens zwei seriell gekoppelte Rohrreaktoren und insbesondere bevorzugt genau zwei seriell gekoppelte Rohrreaktoren eingesetzt. Die Hydrierbedingungen in den eingesetzten Reaktoren können jeweils gleich oder unterschiedlich sein und liegen jeweils in den oben beschriebenen Bereichen.

Wird die Hydrierung an mindestens einem suspendierten Katalysator durchgeführt, liegt die Verweilzeit im Allgemeinen im Bereich von 0,05 bis 50 h, beispielsweise im Bereich von 0,5 bis 50 h, bevorzugt im Bereich von 1 bis 30 h und besonders bevorzugt im Bereich von 1,5 bis 25 h, ganz besonders bevorzugt im Bereich von 1,5 bis 10 h. Dabei ist es unerheblich, ob erfindungsgemäß ein Hauptreaktor und ein Nachreaktor oder zusätzlich weitere Reaktoren eingesetzt werden. Für sämtliche dieser Ausführungsformen liegt die Gesamtverweilzeit in den oben angegebenen Bereichen.

Wird im Rahmen des erfindungsgemäßen Verfahrens die Hydrierung in kontinuierlicher Fahrweise an mindestens einem fest angeordneten Katalysator durchgeführt, so liegt die Katalysatorbelastung (kg Feed/Liter Katalysator x h) im Allgemeinen im Bereich von 0,03 bis 20, bevorzugt im Bereich von 0,05 bis 5 und besonders bevorzugt im Bereich von 0,1 bis 2. Dabei ist es unerheblich, ob erfindungsgemäß ein Hauptreaktor und ein Nachreaktor oder zusätzlich weitere Reaktoren eingesetzt werden. Für sämtliche dieser Ausführungsformen liegt die Gesamtbelastung in den oben angegebenen Bereichen.

Der oder die Nachreaktoren können auf dem gleichen, bei niedrigerem oder auch höherem Temperaturniveau wie der Hauptreaktor betrieben werden. Allgemein liegt die Hydriertemperatur im Hauptreaktor im Bereich von 0 bis 350 °C, bevorzugt im Bereich von 20 bis 300 °C. weiter bevorzugt im Bereich von 50 bis 250 °C und insbesondere bevorzugt im Bereich von 80 bis 220 °C.

Daher betrifft die vorliegenden Erfindung gemäß einer weiteren Ausführungsform ein wie zuvor beschriebenes Verfahren zur Hydrierung einer Zusammensetzung I, wobei die Umsetzung der Zusammensetzung I mit Wasserstoff im Hauptreaktor bei einer Temperatur im Bereich von 0°C bis 350°C durchgeführt wird.

Der Wasserstoffdruck liegt bei der erfindungsgemäßen Hydrierung im Hauptreaktor im Allgemeinen im Bereich von 1 bis 325 bar, bevorzugt im Bereich von 5 bis 300 bar, weiter bevorzugt im Bereich von 10 bis 250 bar und insbesondere bevorzugt im Bereich von 15 bis 150 bar. Dabei ist es vorteilhaft, wenn das Druckniveau im Nachreaktor gleich oder höher ist als im Hauptreaktor.

Die vorliegende Erfindung betrifft daher gemäß einer weiteren Ausführungsform ein wie zuvor beschriebenes Verfahren zur Hydrierung einer Zusammensetzung I, wobei die Umsetzung der Zusammensetzung I mit Wasserstoff im Hauptreaktor bei einem Druck im Bereich von 1 bar bis 325 bar durchgeführt wird.

Das Gemisch, das aus dem Hauptreaktor erhalten wird, enthält Reaktionsprodukt, vorzugsweise umgesetzte organische Verbindung, in einem Umsatzanteil, bezogen auf das gesamte Gemisch, bevorzugt im Bereich von 60 bis 99,9 % und besonders bevorzugt im Bereich von 70 bis 99,5 %. Dieses Gemisch wird, gegebenenfalls nach mindestens einer geeigneten Zwischenbehandlung, dem Nachreaktor zugeführt. Das Gemisch, das aus dem Nachreaktor erhalten wird, enthält Reaktionsprodukt, vorzugsweise umgesetzte organische Verbindung, in einem Anteil bevorzugt im Bereich von mindestens 99,5 %, weiter bevorzugt im Bereich von 99,9 % und insbesondere bevorzugt von 99,99 %.

Im Rahmen der erfindungsgemäßen Hydrierung kann mindestens ein geeignetes Lösungsmittel oder Verdünnungsmittel eingesetzt werden. Als solche sind grundsätzlich sämtliche Lösungs- und Verdünnungsmittel zu nennen, die unter den Hydrierbedingungen nicht hydriert oder anderweitig umgesetzt werden, z.B. Alkohole, Ether, Kohlenwasserstoffe, Wasser, Aromaten oder Ketone. Insbesondere geeignet sind sofern als organische Verbindung Cyclododecadienon in der Zusammensetzung I enthalten ist, unter anderem Toluol oder Cyclododecan.

Gemäß einer bevorzugten Ausführungsform des erfindungsgemäßen Verfahrens wird die Hydrierung ohne Zusatz eines Lösungs- oder Verdünnungsmittels durchgeführt.

Aus der erfindungsgemäßen Hydrierung wird im Allgemeinen ein Gemisch erhalten, das neben Reaktionsprodukt, vorzugsweise umgesetzte organische Verbindung, gegebenenfalls mindestens ein Nebenprodukt und/oder mindestens eine weitere Verbindung enthält, die über beispielsweise ein Gemisch, enthaltend die organische Verbindung, der Hydrierung zugeführt wurde. Aus diesem Gemisch kann das Reaktionsprodukt, vorzugsweise umgesetzte organische Verbindung, über mindestens eine geeignete Methode wie beispielsweise bevorzugt über mindestens eine Destillation abgetrennt werden.

Das erfindungsgemäße Verfahren zeichnet sich durch einen hohen Umsatz aus, was zu einer hohen Reinheit der erhaltenen Produkte führt. Die Katalysatorlebensdauer, insbesondere im Nachreaktor, erhöht sich, die Katalysatoraktivität im Nachreaktor ist durch die geringen CO-Mengen höher, d.h. erfindungsgemäß kann weniger Katalysator eingesetzt werden, wobei im Allgemeinen auch weniger Reaktionsdruck aufzubringen ist.

Die vorliegende Erfindung wird durch die folgenden Beispiele illustriert.

### BEISPIELE

### Vergleichsbeispiel 1 : Hydrierung von Cyclododeca-4,8-dien-1-on zu Cyclododecanon

In einer Reaktorkaskade bestehend aus einem Rohrreaktor mit Flüssigumlauf und einem Nachreaktor wurde Cyclododeca-4,8-dien-1-on kontinuierlich hydriert (30 bar, Temperaturen 130°, jeder Reaktor in Rieselfahrweise, Katalysator 0,2% Pd auf Aluminiumoxid), welches noch ca. 0,5 % mehrfach ungesättigte C12-Aldehyde enthielt. Die gesamte Wasserstoffmenge (2,1 Mol-Äquivalente bezogen auf Edukt, d.h. 5 Mol-% Wasserstoffüberschuss) wurde in den Hauptreaktor eingebracht und zusammen mit dem Produktstrom in den Nachreaktor überführt. Danach wurde das restliche Gas als Abgas ausgeschleust . Bei einer Gesamtkatalysatorbelastung von 0,5 kg Dienon/Liter Katalysator x h betrug der Umsatz 99,8 %, d.h. im Reaktionsaustrag befanden sich noch 0,2 % unumgesetztes Olefin. In einer nachfolgenden Destillation konnte Cyclododecanon in einer Reinheit von 99,4 % erhalten werden, 0,2 % ungesättigtes Cyclododecanon war unerwünschter Nebenbestandteil.

### Beispiel 1 : Hydrierung von Cyclododeca-4,8-dien-1-on zu Cyclododecanon

Vergleichsbeispiel 1 wurde mit dem Unterschied wiederholt, dass der Wasserstoff in den Nachreaktor eingebracht und daraus der verbleibende Wasserstoff in den Hauptreaktor überführt wurde, aus dem das Abgas ausgeschleust wurde. Der Umsatz nach dem Nachreaktor betrug 99,99 %. Bei der Destillation wurde Cyclododecanon in einer Reinheit von 99,6 % erhalten. Unerwünschte ungesättigte Produkte waren nur noch mit einem Gehalt von 100 ppm enthalten.

### Vergleichsbeispiel 2 : Hydrierung von Dehydrolysmeral zu Lysmeral

Analog Vergleichsbeispiel 1 wurde ein Gemisch aus 50 % Methanol, 29 % Dehydrolysmeral, 13 % tert.-Butylbenzaldehyd, 5 % Wasser und 3 % anderer Komponenten wie Propanol, Propanal, verschiedene Ester und Schwersieder wie Natriumsalze hydriert. Als Katalysator wurde 5 % Pd auf Aktivkohle verwendet. Die Hydriertemperatur im Hauptreaktor betrug ca. 80 °C, im Nachreaktor ca. 130 °C, der Reaktionsdruck lag bei 15 bar. Der Wasserstoffüberschuss bezogen auf Dehydrolysmeral betrug 1,05 Moläquivalente. Der Dehydrolysmeral-Umsatz lag bei einer Katalysatorbelastung von ca. 2 kg/Liter Katalysator x h nach dem Nachreaktor bei 93 % und die Lysmeralselektivität bei 91 %.

### Beispiel 2 (Vergleichsbeispiel)

Vergleichsbeispiel 2 wurde analog Beispiel 1 wiederholt. Der Umsatz nach dem Nachreaktor betrug 97 % und die Lysmeralselektivität lag bei 94 %.

## Patentansprüche

1. Verfahren zur Hydrierung einer Zusammensetzung I, enthaltend mindestens einen Aldehyd und Cyclododecadienon, das durch Umsetzung eines Cyclododecatriens mit Distickstoffmonoxid erhalten wurde, mit Wasserstoff in Gegenwart eines Katalysators in mindestens einem Hauptreaktor und mindestens einem Nachreaktor, wobei mindestens 50% des dem Reaktionssystem zugeführten Frischwasserstoffs in mindestens einen Nachreaktor eingespeist werden und wobei mindestens 70% des Abgases des Hauptreaktors ausgeschleust werden.

2. Verfahren nach Anspruch 1, wobei mehr als 50% des in mindestens einem Hauptreaktor eingesetzten Wasserstoffs aus dem Abgas des Nachreaktors stammen.

3. Verfahren nach Anspruch 1 oder 2, wobei Wasserstoff in einem Überschuss von 0,1 bis 50 Mol-% eingesetzt wird.

4. Verfahren nach einem der Ansprüche 1 bis 3, wobei der mindestens eine Aldehyd ausgewählt ist aus der Gruppe bestehend aus C1- bis C20-Aldehyden.

5. Verfahren nach einem der Ansprüche 1 bis 4, wobei im Hauptreaktor mindestens 60% der Zusammensetzung I umgesetzt werden.

6. Verfahren nach einem der Ansprüche 1 bis 5, wobei mindestens 60% des dem Reaktionssystem zugeführten Frischwasserstoffs in mindestens einen Nachreaktor eingespeist werden.

7. Verfahren nach einem der Ansprüche 1 bis 6, wobei Wasserstoff nur in Form von im Produktstrom des Hauptreaktors gelöstem Wasserstoff von mindestens einem Hauptreaktor zu mindestens einem Nachreaktor gelangt.

8. Verfahren nach einem der Ansprüche 1 bis 7, wobei der Katalysator als Aktivmetall Pd, Pt, Ru oder Ni enthält.

9. Verfahren nach einem der Ansprüche 1 bis 8, wobei die Umsetzung der Zusammensetzung I mit Wasserstoff im Hauptreaktor bei einer Temperatur im Bereich von 0°C bis 350°C durchgeführt wird.

10. Verfahren nach einem der Ansprüche 1 bis 9, wobei die Umsetzung der Zusammensetzung I mit Wasserstoff im Hauptreaktor bei einem Druck im Bereich von 1 bar bis 325 bar durchgeführt wird.

## Claims

1. A process for hydrogenating a composition I, comprising at least one aldehyde and cyclododecadienone which has been obtained by reacting a cyclododecatriene with dinitrogen monoxide, with hydrogen in the presence of a catalyst in at least one main reactor and at least one postreactor, wherein at least 50% of the fresh hydrogen fed to the reaction system is fed into at least one postreactor and wherein at least 70% of the offgas of the main reactor is discharged.

2. The process according to claim 1, wherein more than 50% of the hydrogen used in at least one main reactor stems from the offgas of the postreactor.

3. The process according to claim 1 or 2, wherein hydrogen is used in an excess of from 0.1 to 50 mol%.

4. The process according to any of claims 1 to 3, wherein the at least one aldehyde is selected from the group consisting of C1- to C20-aldehydes.

5. The process according to any of claims 1 to 4, wherein at least 60% of composition I is reacted in the main reactor.

6. The process according to any of claims 1 to 5, wherein at least 60% of the fresh hydrogen fed to the reaction system is fed into at least one postreactor.

7. The process according to any of claims 1 to 6, wherein hydrogen passes from at least one main reactor to at least one postreactor only in the form of hydrogen dissolved in the product stream of the main reactor.

8. The process according to any of claims 1 to 7, wherein the catalyst comprises, as an active metal, Pd, Pt, Ru or Ni.

9. The process according to any of claims 1 to 8, wherein the reaction of composition I with hydrogen in the main reactor is carried out at a temperature in the range from 0°C to 350°C.

10. The process according to any of claims 1 to 9, wherein the reaction of composition I with hydrogen in the main reactor is carried out at a pressure in the range from 1 bar to 325 bar.

## Revendications

1. Procédé d'hydrogénation d'une composition I, contenant au moins un aldéhyde et une cyclododécadiénone, qui a été obtenue par réaction d'un cyclododécatriène avec du monoxyde de diazote, avec de l'hydrogène en présence d'un catalyseur dans au moins un réacteur principal et au moins un réacteur secondaire, au moins 50 % de l'hydrogène frais introduit dans le système réactionnel étant introduit dans au moins un réacteur secondaire et au moins 70 % du gaz d'échappement du réacteur principal étant déchargé.

2. Procédé selon la revendication 1, dans lequel plus de 50 % de l'hydrogène utilisé dans au moins un réacteur principal provient du gaz d'échappement du réacteur secondaire.

3. Procédé selon la revendication 1 ou 2, dans lequel l'hydrogène est utilisé en un excès de 0,1 à 50 % en moles.

4. Procédé selon l'une quelconque des revendications 1 à 3, dans lequel le ou les aldéhydes sont choisis dans le groupe constitué par les aldéhydes en C1 à C20.

5. Procédé selon l'une quelconque des revendications 1 à 4, dans lequel au moins 60 % de la composition I est mise en réaction dans le réacteur principal.

6. Procédé selon l'une quelconque des revendications 1 à 5, dans lequel au moins 60 % de l'hydrogène frais introduit dans le système réactionnel est introduit dans au moins un réacteur secondaire.

7. Procédé selon l'une quelconque des revendications 1 à 6, dans lequel l'hydrogène n'atteint au moins un réacteur secondaire à partir d'au moins un réacteur principal que sous la forme d'hydrogène dissous dans le courant de produits du réacteur principal.

8. Procédé selon l'une quelconque des revendications 1 à 7, dans lequel le catalyseur contient Pd, Pt, Ru ou Ni en tant que métal actif.

9. Procédé selon l'une quelconque des revendications 1 à 8, dans lequel la réaction de la composition I avec de l'hydrogène dans le réacteur principal est réalisée à une température dans la plage allant de 0 °C à 350 °C.

10. Procédé selon l'une quelconque des revendications 1 à 9, dans lequel la réaction de la composition I avec de l'hydrogène dans le réacteur principal est réalisée à une pression dans la plage allant de 1 bar à 325 bar.
